# FASCICULE DE BREVET EUROPEEN

(11) **EP 3 795 253 B1**
(45) Date de publication et mention de la délivrance du brevet: **10.04.2024**
(21) Numéro de dépôt: 20193535.0
(22) Date de dépôt: 31.08.2020
(51) Int. Cl.: B01L 3/00, G01N 35/00, B01L 9/00

(54) **EQUIPEMENT POUR LA TRAÇABILITÉ DE PRÉLÈVEMENTS BIOLOGIQUES**
ANLAGE FÜR DIE RÜCKVERFOLGBARKEIT VON BIOLOGISCHEN PROBEN
EQUIPMENT FOR THE TRACEABILITY OF BIOLOGICAL SAMPLES

(30) Priorité: 19.09.2019 FR 1910339
(43) Date de publication de la demande: 24.03.2021
(73) Titulaire: Dreampath Diagnostics, 67100 Strasbourg (FR)
(72) Inventeur: JORDAN NAVAS, Pablo, 28005 Madrid (ES); WILHELM, Valérie, 67114 Eschau (FR)
(74) Mandataire: Hugues, Catherine

(56) Documents cités:
- WO-A1-2018/190055
- US-A- 4 430 299
- US-A- 5 320 808
- US-A1- 2015 080 259
- US-A1- 2016 178 653

## Description

La présente invention a pour objet un équipement pour la traçabilité de prélèvements biologiques placés dans des cassettes d'inclusion.

Dans le cadre de la prise en charge médicale d'un patient ou celui de travaux de recherche dans le domaine humain, animal ou végétal, des prélèvements tissulaires ou cellulaires sont couramment effectués en vue d'une analyse histologique et/ou moléculaire, destinée ou non à l'établissement d'un diagnostic.

Afin de permettre une parfaite visualisation au microscope des structures cellulaires ou tissulaires analysées, de tels prélèvements font l'objet d'une préparation préalable comportant différentes étapes.

De manière résumée, au cours de ces dernières, les échantillons frais, initialement conservés dans des pots d'échantillonnage remplis de formol, sont placés dans des cassettes d'inclusion ajourées, pour subir une phase de déshydratation, puis une étape d'inclusion en paraffine conduisant à des blocs d'échantillons biologiques. Des coupes fines et régulières, prélevées à partir de ces derniers, sont ensuite placées sur des lames porte-objet, subissent encore différents traitements destinés à colorer les éléments structurels à observer, et sont enfin recouvertes d'une résine puis d'une lamelle permettant leur lecture au microscope et assurant leur protection.

Toutes ces étapes de préparation des échantillons biologiques en vue de leur analyse supposent de nombreuses manipulations au cours desquelles des mesures pour en assurer la traçabilité, doivent être mises en oeuvre.

Des solutions ont d'ores et déjà été proposées à cet effet, en particulier pour assurer une traçabilité automatisée d'échantillons biologiques stockés dans des blocs de paraffine ou sur des lames porte-objet.

Toutefois, il a été constaté qu'à ce jour, aucune solution rationnelle n'est encore disponible pour gérer de manière efficace et fiable les échantillons biologiques placés dans des cassettes d'inclusion en vue de leur déshydratation.

De fait, les équipements existants sont développés pour des applications bien spécifiques, et conviennent selon le cas pour le suivi des blocs de paraffine uniquement ou pour celui des lames porte-objet uniquement.

En somme, aucun appareil dédié à un suivi de cassettes d'inclusion n'a pour le moment été proposé, de sorte que les opérations nécessaires à la traçabilité d'échantillons biologiques stockés dans de tels contenants doivent, à ce stade, encore être réalisées manuellement.

De manière classique, chaque échantillon biologique se voit attribuer un numéro de référence unique, fourni par le système informatique de l'établissement ayant procédé à son prélèvement. Ce numéro le lie au patient dont il provient et va le suivre jusqu'à sa destruction, qu'il soit stocké dans un pot d'échantillonnage, une cassette d'inclusion, un bloc de paraffine ou sur une lame porte-objet, sous forme de données encodées, de type code à barre, code matriciel, puce RFID, apposées sur ces derniers, au fur et à mesure de leur préparation.

Actuellement, lors de leur prise en charge par les services d'analyse compétents, les prélèvements biologiques fraîchement prélevés sont enregistrés dans une base de données, à partir d'une lecture manuelle des données encodées apposées sur les pots de formol les contenant. Plus précisément, au terme de cette étape, la base de données contient pour chaque échantillon des informations telles que l'identité du patient, la nature du prélèvement et le nombre de cassettes à préparer à partir de celui-ci. Les prélèvements sont ensuite extraits l'un après l'autre de leur pot, soumis à un premier examen visuel, puis placés individuellement dans des cassettes ajourées munies de leurs données d'identification et permettant de les exposer à différents traitements destinés à remplacer l'eau contenue dans leurs cellules par une résine. Les étapes de ce processus sont couramment appliquées simultanément à une pluralité de prélèvements biologiques au moyen d'appareils dédiés, appelés « tissue processor ». A cet effet, les cassettes dans lesquelles ils sont contenus sont regroupées dans des paniers fournis avec ces mêmes appareils, facilitant leur manipulation. A des fins de traçabilité, lorsque les paniers sont remplis de cassettes, il est d'usage de vérifier manuellement, à l'aide de la base de données renseignée initialement, si le nombre de cassettes présentes dans chaque panier pour « tissue processor » correspond bien au nombre de cassettes susceptibles d'avoir été préparées. En cas de différence, une recherche d'erreur peut ainsi être effectuée, afin de retrouver le cas échéant une cassette oubliée sur une paillasse avec son contenu, ou de s'assurer que l'ensemble des prélèvements réceptionnés par le laboratoire ont été placés dans les cassettes et ont par conséquent été traités. Le délai de conservation des échantillons dans le formol étant très court, un tel contrôle s'avère en effet indispensable pour éviter toute perte de matière intempestive. Des exemples d'équipements permettant de relever des données encodées apposées sur des cassettes rangées dans des paniers sont décrits par les documents WO 2018/190055A1 et US5320808A.

De toute évidence, une telle manière manuelle de procéder est particulièrement fastidieuse et chronophage, dans la mesure où elle suppose plusieurs manipulations de chaque prélèvement biologique pour relever et enregistrer ses données d'identification lors des étapes successives mentionnées ci-dessus, et comparer entre elles les informations relatives à ces mêmes étapes figurant dans la base de données.

De plus, en raison du vieillissement de la population, et de la croissance du taux d'incidence de certaines maladies, telles que notamment le cancer, le nombre d'échantillons arrivant quotidiennement dans les laboratoires d'histopathologie ne cesse d'augmenter. Une telle situation conduit inévitablement à un alourdissement de la charge de travail des personnels de laboratoire, qui ne peuvent que déplorer l'obsolescence des méthodes de traçabilité manuelles dont ils disposent actuellement pour accomplir une partie de leurs tâches.

En conséquence, la présente invention a pour objectif de proposer une solution permettant d'améliorer la productivité de l'ensemble du personnel susceptible d'intervenir lors des phases de préparation, d'analyse et de rangement de prélèvements tissulaires ou cellulaires grâce à une automatisation de la traçabilité d'échantillons biologiques au cours de l'ensemble du processus de préparation et d'analyse qu'ils subissent, y compris durant l'étape de déshydratation, afin de réduire la charge de travail des opérateurs, éviter tout risque d'erreur, de perte de matériel et pour garantir l'établissement de diagnostics fiables attribués au bon patient.

D'autre part, l'invention a également pour but de proposer un équipement compatible avec l'utilisation de produits potentiellement toxiques et/ou corrosifs, et présentant une structure permettant de limiter l'exposition à ces mêmes produits des opérateurs amenés à le manipuler et de protéger ses éléments constitutifs.

A cet effet, la présente invention concerne un équipement pour la traçabilité de prélèvements biologiques placés dans des cassettes d'inclusion munies d'informations d'identification sous forme de données encodées et destinées à être rangées les unes à côté des autres dans des logements ménagés au sein de paniers dont la forme générale est susceptible de varier d'un panier à un autre, ledit équipement comportant :
- au moins un réceptacle conçu apte à accueillir au moins un panier,
- au moins un appareil de lecture des données encodées apposées sur des cassettes disposées dans au moins un panier placé dans le réceptacle, ledit appareil étant délimité par un fond, une paroi périphérique et une paroi supérieure et étant pourvu d'au moins une ouverture d'accès à au moins une cavité interne conformée pour accueillir le réceptacle et équipée de moyens de lecture de données encodées,
- des moyens de traitement informatique des données lues par les moyens de lecture, et
- des moyens d'identification de la forme du/des panier(s) placé(s) dans le réceptacle.

Conformément à une caractéristique de l'équipement selon l'invention, le réceptacle est subdivisé en plusieurs compartiments de différentes formes, conformés chacun pour réceptionner un panier d'une forme donnée, tandis que l'appareil de lecture est équipé de moyens de détection conçus aptes à détecter la présence ou, le cas échéant, l'absence, d'un panier dans au moins un desdits compartiments dudit réceptacle et à transmettre l'information correspondante à des moyens de pilotage des moyens de lecture de données encodées.

D'autre part, il est également prévu que le réceptacle comporte de préférence un fond ajouré.

Selon une caractéristique additionnelle, la cavité de l'appareil peut comporter des moyens support d'un réceptacle, conformés pour permettre une inclinaison du fond du réceptacle par rapport au fond de l'appareil. L'inclinaison a pour avantage de favoriser l'écoulement du formol imprégnant l'échantillon contenu dans les cassettes, et par conséquent de faciliter la lecture des données apposées sur ces dernières.

A cet effet, une variante de réalisation a été imaginée dans laquelle la paroi périphérique de l'appareil comporte deux parois opposées munies de rails formant chacun un angle α, de préférence compris entre 10° et 80°, avec le fond de l'appareil, lesdits rails étant conçus aptes à coopérer avec des coulisses que comporte ledit réceptacle.

De plus, l'équipement selon l'invention se caractérise encore en ce qu'il peut comporter un bac conformé pour reposer sur son fond.

Par ailleurs, une caractéristique additionnelle de l'équipement selon l'invention est définie par le fait que les moyens de lecture des données encodées que comporte ledit appareil de lecture sont conçus aptes à résister à une atmosphère humide.

Au moins un ventilateur installé dans la cavité dudit appareil peut en outre être prévu, en vue d'évacuer activement les vapeurs de formol risquant d'empêcher la lecture des données encodées.

Conformément à l'invention, les moyens de lecture des données encodées peuvent comporter un scanner lumineux et/ou un lecteur radio de type RFID.

Les dessins annexés illustrent l'invention :
[Fig.1] représente des vues schématiques de paniers pour stocker des cassettes d'inclusion lors de l'étape de déshydratation des prélèvements biologiques, présentant différentes structures,
[Fig.2] représente une vue de dessus d'une variante de réalisation d'un réceptacle selon l'invention, et

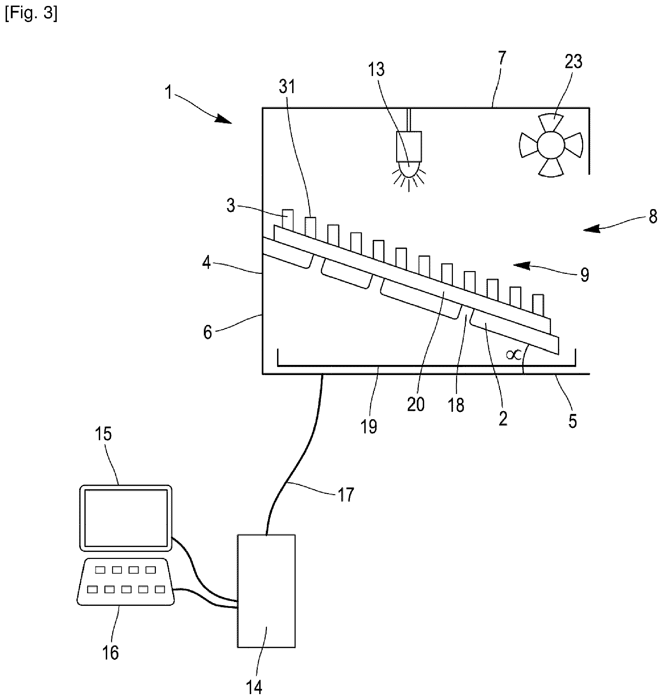

[Fig. 3] illustre une vue en coupe schématique de l'équipement selon l'invention.

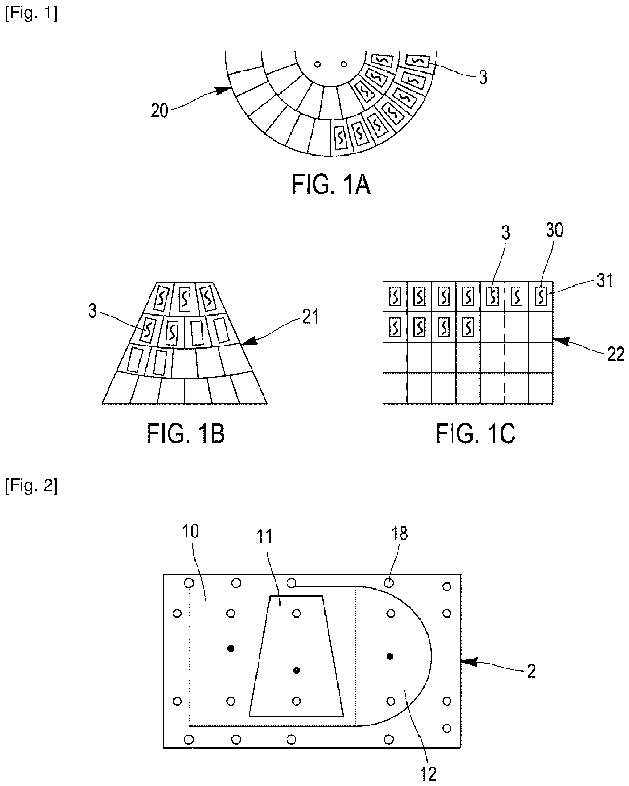

En référence à ces dessins, dans la variante de réalisation illustrée, l'équipement 1 selon l'invention comporte un réceptacle 2 à caractère universel conçu apte à accueillir différents types de paniers 20, 21, 22 pour cassettes d'inclusion 3. De manière classique, de tels paniers 20, 21, 22 sont fournis avec les « tissue processor » utilisés pour déshydrater les échantillons, et présentent une structure dont la forme est susceptible de varier, d'un modèle de panier à un autre. En somme la forme des paniers est définie par leur fabricant, et peut par exemple correspondre à une forme en demi-lune (cf. fig. 1A), une forme trapézoïdale (cf. fig. 1B), une forme parallélépipédique (cf. fig. 1C), ou toute autre forme permettant de ranger de manière ordonnancée une pluralité de cassettes d'inclusion 3. Tel que visible aux figures 1A à 1C, ces dernières sont généralement positionnées dans des logements individuels que comportent les paniers 20, 21, 22, de manière telle que leur face 30 munie des données d'identification encodées 31 soit visible et de préférence orientée vers le haut.

En référence à la figure 2, il a été imaginé dans le cadre de l'invention, de concevoir le réceptacle 2 de manière telle qu'il comporte différents compartiments de différentes formes, dédiés chacun à la réception d'un panier 20, 21, 22 de structure ou de forme donnée. Ainsi, dans l'exemple illustré à la figure 2, le réceptacle 2 se présente sous la forme d'un plateau, dont le fond présente de préférence une pluralité de lumières 18, comportant trois compartiments 10, 11, 12 dédiés à la réception respectivement d'un panier 22 de forme parallélépipédique, d'un panier 21 de forme trapézoïdale et d'un panier 20 en forme de demi-lune. Les zones de réception 10 et 11 se chevauchant, il va de soi qu'un tel réceptacle n'est pas en mesure d'accueillir simultanément un panier 22 de forme parallélépipédique et un panier 21 de forme trapézoïdale. Par contre, il est tout à fait adapté à la réception simultanée d'un panier 22 de forme parallélépipédique ou d'un panier 21 de forme trapézoïdale et d'un panier 20 en forme de demi-lune. En d'autres termes, un tel réceptacle 2 pourra accueillir selon le cas un seul panier 10, ou 11, ou 12 ou deux paniers 10 et 12 ou 11 et 12. Bien entendu, un agencement des compartiments autre que celui que comporte le réceptacle 2 de l'exemple illustré peut également être prévu.

Par ailleurs, l'équipement 1 comporte également un appareil 4 de lecture des données encodées apposées sur des cassettes 3 disposées dans au moins un des paniers 20, 21, 22 placé dans un réceptacle 2. Plus précisément, l'appareil 4 est délimité par un fond 5, une paroi périphérique 6, et une paroi supérieure 7. Il est pourvu d'au moins une ouverture 8 d'accès à au moins une cavité interne 9 conformée pour accueillir un réceptacle 2 et équipée de moyens 13 de lecture de données encodées, tels que par exemple un scanner lumineux ou un lecteur radio de type « RFID ». Des moyens de traitement informatique des données lues par les moyens de lecture 13, tels qu'un ordinateur 14 comprenant un écran 15 et un clavier 16 sont par ailleurs reliés à l'appareil 4 par une liaison filaire ou non filaire 17.

Dans la variante de réalisation illustrée, l'appareil de lecture 4 est encore équipé de moyens de détection (non illustrés) de la présence d'au moins un panier 20, 21, 22 dans le réceptacle 2, et conçus aptes à transmettre l'information correspondante à des moyens de pilotage des moyens de lecture de données encodées 31. En somme, une telle caractéristique permet de programmer automatiquement, suite à une simple détection de la présence d'un panier dans un compartiment donné, les moyens de lecture de manière telle qu'ils soient à même de lire convenablement les données d'identification 31 apposées sur des cassettes d'inclusion 3 disposées selon le cas dans un panier 20 en forme de demi-lune, ou dans un panier 21 de structure trapézoïdale, ou dans un panier 22 de structure parallélépipédique. Grâce à une telle structure, l'équipement 1 permet d'automatiser la lecture des données encodées 31 que comportent les cassettes 3 et de procéder à la vérification de l'intégralité du contenu d'un ou deux paniers 20, 21, ou 22 simultanément en une seule étape, quelle que soit la forme générale de tels paniers.

D'autre part, dans la variante de réalisation illustrée, la paroi périphérique 6 de l'appareil 4 comporte deux parois opposées munies de rails formant chacun un angle α, de préférence compris entre 10° et 80°, avec le fond 5 de l'appareil 4 et conçus aptes à coopérer avec des coulisses que comporte le réceptacle 2. Une telle structure favorise l'éventuel écoulement de formol, dont les prélèvements biologiques contenus dans les cassettes d'inclusion 3 peuvent encore être imprégnés, au travers des lumières 18 du réceptacle 2. Un bac 19 amovible, conformé pour reposer sur le fond 5 de l'appareil 4, permet par ailleurs de récupérer et d'évacuer tout liquide s'écoulant du réceptacle 4, afin d'éviter toute stagnation de produit toxique dans l'appareil 4 et favoriser une lecture convenable des données encodées 31.

A ce propos, il convient de noter qu'afin d'améliorer la longévité de l'appareil 4 et également favoriser une lecture convenable des données encodées 31, au moins un ventilateur 23 est installé dans la cavité 9, tandis que les moyens de lecture 13 des données encodées sont préférentiellement conformés pour résister à une atmosphère humide et potentiellement corrosive.j

## Revendications

1. Equipement (1) pour la traçabilité de prélèvements biologiques placés dans des cassettes d'inclusion (3) munies d'informations d'identification sous forme de données encodées (31) et destinées à être rangées les unes à côté des autres dans des logements ménagés au sein de paniers (20, 21, 22) dont la forme générale est susceptible de varier d'un panier à un autre, ledit équipement comportant :
- au moins un réceptacle (2) conçu apte à accueillir au moins un panier (20, 21, 22),
- au moins un appareil (4) de lecture des données encodées (31) apposées sur des cassettes (3) disposées dans au moins un panier (20, 21, 22) placé dans le réceptacle (2), ledit appareil (4) étant délimité par un fond (5), une paroi périphérique (6) et une paroi supérieure (7) et étant pourvu d'au moins une ouverture d'accès (8) à au moins une cavité interne (9) conformée pour accueillir le réceptacle (2) et équipée de moyens de lecture (13) de données encodées (31),
- des moyens de traitement informatique (14) des données lues par les moyens de lecture (13), et
- des moyens d'identification de la forme du/des panier(s) (20, 21, 22) placé(s) dans le réceptacle (2),
**caractérisé en ce que** le réceptacle (2) est subdivisé en plusieurs compartiments (10, 11, 12) de différentes formes, conformés chacun pour réceptionner un panier (20, 21, 22) d'une forme donnée, tandis que l'appareil de lecture (4) est équipé de moyens de détection conçus aptes à détecter la présence ou, le cas échéant, l'absence, d'un panier (20, 21, 22) dans au moins un desdits compartiments (10, 11, 12) dudit réceptacle (4) et à transmettre l'information correspondante à des moyens de pilotage des moyens de lecture (13) de données encodées (31).

2. Equipement (1) selon la revendication 1, **caractérisé en ce que** le fond du réceptacle (2) est ajouré.

3. Equipement (1) selon l'une quelconque des
revendications précédentes, **caractérisé en ce que** la cavité (9) de l'appareil (4) comporte des moyens support d'un réceptacle (2), conformés pour permettre une inclinaison du fond du réceptacle (2) par rapport au fond (5) de l'appareil (4).

4. Equipement (1) selon la revendication 3, **caractérisé en ce que** la paroi périphérique (6) de l'appareil (4) comporte deux parois opposées munies de rails formant chacun un angle α avec le fond (5) de l'appareil (4) et conçus aptes à coopérer avec des coulisses que comporte ledit réceptacle (2).

5. Equipement (1) selon la revendication 4, **caractérisé en ce que** l'angle α est compris entre 10° et 80°.

6. Equipement (1) selon l'une quelconque des
revendications précédentes, **caractérisé en ce que** l'appareil (4) comporte un bac amovible (19) conformé pour reposer sur son fond (5).

7. Equipement (1) selon l'une quelconque des
revendications précédentes, **caractérisé en ce que** les moyens de lecture (13) des données encodées (31) dudit appareil de lecture (4) sont conçus aptes à résister à une atmosphère humide.

8. Equipement (1) selon l'une quelconque des
revendications précédentes, **caractérisé en ce que** ledit appareil (4) est équipé d'au moins un ventilateur (23) installé dans ladite cavité (9).

9. Equipement (1) selon l'une quelconque des
revendications précédentes, **caractérisé en ce que** les moyens de lecture (13) de données encodées (31) sont définis par un scanner lumineux, et/ou un lecteur radio de type « RFID ».

## Patentansprüche

1. Ausrüstung (1) für die Rückverfolgbarkeit von biologischen Proben, die in Einbettungskassetten (3) eingesetzt sind, die mit Identifikationsinformationen in Form von codierten Daten (31) versehen und dazu bestimmt sind, nebeneinander in Einlassungen eingereiht zu werden, die innerhalb von Körben (20, 21, 22) angebracht sind, deren allgemeine Form imstande ist, von einem Korb zu einem anderen zu variieren, wobei die Ausrüstung aufweist:
- mindestens einen Behälter (2), der geeignet entworfen ist, um mindestens einen Korb (20, 21, 22) unterzubringen,
- mindestens eine Einrichtung (4) zum Lesen von codierten Daten (31), die auf den Kassetten (3) angefügt sind, die in mindestens einem Korb (20, 21, 22) angeordnet sind, der in den Behälter (2) eingesetzt ist, wobei die Einrichtung (4) durch einen Boden (5), eine Umfangswand (6) und eine obere Wand (7) begrenzt ist und mit mindestens einer Zugangsöffnung (8) zu mindestens einem inneren Hohlraum (9) vorgesehen ist, der zum Unterbringen des Behälters (2) entworfen und mit Mitteln (13) zum Lesen von codierten Daten (31) ausgerüstet ist,
- Mittel (14) zum computergestützten Verarbeiten der Daten, die durch die Lesemittel (13) gelesen werden, und
- Mittel zum Identifizieren der Form des/der Korbs/Körbe (20, 21, 22), der/die in den Behälter (2) eingesetzt ist/sind, **dadurch gekennzeichnet, dass** der Behälter (2) in mehrere Fächer (10, 11, 12) mit unterschiedlichen Formen unterteilt ist, die jeweils zum Aufnehmen eines Korbs (20, 21, 22) mit einer gegebenen Form entworfen sind, während die Leseeinrichtung (4) mit Erfassungsmitteln ausgerüstet ist, die geeignet entworfen sind, um das Vorhandensein oder gegebenenfalls die Abwesenheit eines Korbs (20, 21, 22) in mindestens einem der Fächer (10, 11, 12) des Behälters (4) zu erfassen, und um die entsprechende Information an Mittel zum Steuern der Mittel (13) zum Lesen von codierten Daten (31) zu übertragen.

2. Ausrüstung (1) nach Anspruch 1, **dadurch gekennzeichnet, dass** der Boden des Behälters (2) gelocht ist.

3. Ausrüstung (1) nach einem der vorstehenden Ansprüche,
**dadurch gekennzeichnet, dass** der Hohlraum (9) der Einrichtung (4) Mittel zum Tragen eines Behälters (2) umfasst, die zum Ermöglichen einer Neigung des Bodens des Behälters (2) in Bezug auf den Boden (5) der Einrichtung (4) angepasst sind.

4. Ausrüstung (1) nach Anspruch 3, **dadurch gekennzeichnet, dass** die Umfangswand (6) der Einrichtung (4) zwei gegenüberliegende Wände aufweist, die mit Schienen versehen sind, die jeweils einen Winkel α mit dem Boden (5) der Einrichtung (4) ausbilden und geeignet entworfen sind, um mit Gleitführungen zusammenzuwirken, die der Behälter (2) aufweist.

5. Ausrüstung (1) nach Anspruch 4, **dadurch gekennzeichnet, dass** der Winkel α zwischen 10° und 80° liegt.

6. Ausrüstung (1) nach einem der vorstehenden Ansprüche,
**dadurch gekennzeichnet, dass** die Einrichtung (4) einen abnehmbaren Trog (19) aufweist, der zum Ruhen auf seinem Boden (5) angepasst ist.

7. Ausrüstung (1) nach einem der vorstehenden Ansprüche,
**dadurch gekennzeichnet, dass** die Mittel (13) zum Lesen der codierten Daten (31) der Leseeinrichtung (4) geeignet entworfen sind, um einer feuchten Atmosphäre standzuhalten.

8. Ausrüstung (1) nach einem der vorstehenden Ansprüche,
**dadurch gekennzeichnet, dass** die Einrichtung (4) mit mindestens einem Ventilator (23) ausgerüstet ist, der in dem Hohlraum (9) installiert ist.

9. Ausrüstung (1) nach einem der vorstehenden Ansprüche,
**dadurch gekennzeichnet, dass** die Mittel (13) zum Lesen von codierten Daten (31) durch einen Lichtscanner und/oder einen Funkleser der Art "RFID" definiert sind.

## Claims

1. Equipment (1) for the traceability of biological samples placed in embedding cassettes (3) provided with identification information in the form of encoded data (31) and intended to be stored next to one another in housings provided within baskets (20, 21, 22) of which the general shape is likely to vary from one basket to another, said equipment comprising:
- at least one receptacle (2) designed to accommodate at least one basket (20, 21, 22),
- at least one apparatus (4) for reading encoded data (31) affixed to cassettes (3) arranged in at least one basket (20, 21, 22) placed in the receptacle (2), said apparatus (4) being delimited by a bottom (5), a peripheral wall (6) and an upper wall (7) and being provided with at least one access opening (8) having at least one internal cavity (9) shaped to accommodate the receptacle (2) and equipped with means (13) for reading encoded data (31),
- means (14) for computer processing data read by the reading means (13), and
- means for identifying the shape of the basket(s) (20, 21, 22) placed in the receptacle (2), **characterized in that** the receptacle (2) is subdivided into multiple compartments (10, 11, 12) of different shapes, each shaped to receive a basket (20, 21, 22) of a given shape, while the reading apparatus (4) is equipped with detection means designed to be able to detect the presence or, if necessary, the absence of a basket (20, 21, 22) in at least one of said compartments (10, 11, 12) of said receptacle (4) and to transmit the corresponding information to means for controlling the means (13) for reading encoded data (31).

2. Equipment (1) according to claim 1, **characterized in that** the bottom of the receptacle (2) is perforated.

3. Equipment (1) according to either of the preceding claims,
**characterized in that** the cavity (9) of the apparatus (4) comprises means for supporting a receptacle (2), shaped to allow the bottom of the receptacle (2) to incline relative to the bottom (5) of the apparatus (4).

4. Equipment (1) according to claim 3, **characterized in that** the peripheral wall (6) of the apparatus (4) comprises two opposite walls provided with rails each forming an angle a with the bottom (5) of the apparatus (4) and designed to be able to engage with slides comprised in said receptacle (2).

5. Equipment (1) according to claim 4, **characterized in that** the angle a is between 10° and 80°.

6. Equipment (1) according to any of the preceding claims,
**characterized in that** the apparatus (4) comprises a removable tray (19) shaped to rest on its bottom (5).

7. Equipment (1) according to any of the preceding claims,
**characterized in that** the means (13) for reading the encoded data (31) of said reading apparatus (4) are designed to be able to withstand a humid atmosphere.

8. Equipment (1) according to any of the preceding claims,
**characterized in that** said apparatus (4) is equipped with at least one fan (23) installed in said cavity (9).

9. Equipment (1) according to any of the preceding claims,
**characterized in that** the means (13) for reading encoded data (31) are defined by a light scanner, and/or a "RFID" radio reader.
